# EUROPÄISCHE PATENTSCHRIFT

(11) **EP 1 926 831 B1**
(45) Veröffentlichungstag und Bekanntmachung des Hinweises auf die Patenterteilung: **22.06.2011**
(21) Anmeldenummer: 06793404.2
(22) Anmeldetag: 11.09.2006
(51) Int. Cl.: C12Q 1/68

(54) **VERFAHREN ZUR AKTIVIERUNG EINER NUKLEINSÄURE FÜR EINE POLYMERASE-REAKTION**
METHOD FOR ACTIVATING A NUCLEIC ACID FOR A POLYMERASE REACTION
PROCEDE POUR ACTIVER UN ACIDE NUCLEIQUE POUR UNE REACTION POLYMERASE

(30) Priorität: 09.09.2005 EP 05019665
(43) Veröffentlichungstag der Anmeldung: 04.06.2008
(73) Patentinhaber: Qiagen GmbH, 40724 Hilden (DE)
(72) Erfinder: KORFHAGE, Christian, 40764 Langenfeld (DE); LÖFFERT, Dirk, 40589 Düsseldorf (DE)
(86) Internationale Anmeldenummer: PCT/EP2006/066223
(87) Internationale Veröffentlichungsnummer: WO 2007/028833

(56) Entgegenhaltungen:
- WO-A-03/072809
- WO-A2-2004/058987
- US-A- 6 033 881
- US-A1- 2004 161 742
- SAMBROOK, FRITSCH, MANIATIS: "Molecular Cloning - A LABORATORY MANUAL" 1989, COLD SPRING HARBOR LABORATORY PRESS , USA , XP002361772 Seite 5.34 - Seite 5.49
- SUZUKI TOSHINORI ET AL: "Mechanistic studies on depurination and apurinic site chain breakage in oligodeoxyribonucleotides" NUCLEIC ACIDS RESEARCH, Bd. 22, Nr. 23, 1994, Seiten 4997-5003, XP002361770 ISSN: 0305-1048
- newton, graham: "PCR", 1994, Spektrum Akademischer Verlag, Heidelberg page 60,
- NEWTON, GRAHAM: 'PCR', 1994, SPEKTRUM AKADEMISCHER VERLAG, HEIDELBERG Seite 60

## Beschreibung

Die vorliegende Erfindung betrifft ein Verfahren zur Aktivierung einer Nukleinsäure, insbesondere einer Desoxyribonukleinsäure (DNA), für eine Polymerase-Reaktion, insbesondere eine Strand-Displacement-Reaktion (Strangverdrängungsreaktion).

Unter einer Polymerase-Reaktion im Sinne der Erfindung wird die Polymerase-Aktivität einer Nukleinsäure-Polymerase verstanden, also das Polymerisieren von Nukleotiden an ein freies 3'-OH-Ende wobei der komplementäre Strang als Template dient. Dabei kann ein eventuell 3' von dem freien 3'-OH-Ende liegender Strang entweder verdrängt werden (Strand-Displacement-Reaktion, siehe unten) oder dieser durch eine 5'-3'-Exonuklease-Aktivität der Polymerase auch abgebaut und durch den neu synthetisierten Strang ersetzt werden (beispielsweise in einer Nick-Displacement-Reaktion).

Aus der US-Patentanmeldung Nr. 2004/0161742 A1 ist ein Verfahren zur Amplifikation einer Nukleinsäurezielsequenz bekannt. Bei dem Verfahren werden DNA-Polymerase und eine Zielprobe mit einem Set von Primern in Kontakt gebracht und die Zielprobe unter Bedingungen inkubiert, welche die Replikation der Zielsequenz fördern, wobei die Zielsequenz keinen denaturierenden Bedingungen unterworfen wird. Die Replikation der Zielsequenz führt zu replizierten Strängen, wobei während der Replikation mindestens einer der replizierten Stränge von der Zielsequenz durch Strangverdrängungsreplikation eines anderen replizierten Strangs entfernt wird.

Die US-Patentschrift Nr. 6,033,881 beschreibt ein Verfahren zur exponentiellen Amplifikation einer Nukleinsäure in einem Reaktionsgemisch mittels mindestens eines Enzyms. Das Reaktionsgemisch enthält eine zu amplifizierende Nukleinsäure sowie Oligonukleotide, welche Basensequenzen aufweisen, die im wesentlichen komplementär zu den Basensequenzen der zu amplifizierenden Nukleinsäure sind. Die Oligonukleotide können, unter geeigneten Umständen, Startpunkte und/oder chemische Oligonukleotidbildungsblöcke für eine Nukleinsäuresynthese bilden. Das Verfahren umfasst den Schritt der Bildung eines Reaktionsprodukts enthaltend das Oligonukleotid, welches im wesentlichen komplementär zu der zu amplifizierenden Nukleinsäure ist. Das Reaktionsprodukt wird dann in einem weiteren Schritt mit Oligonukleotiden umgesetzt, so dass ein zusätzliches Reaktionsprodukt gebildet und die zu amplifizierende Nukleinsäure amplifiziert wird. Die Schritte werden bei einer Temperatur durchgeführt, welche die Doppelstrangform des Reaktionsprodukts destabilisiert, wobei die Temperatur während der Amplifikation im wesentlichen isothermal gehalten wird.

In Sambrook, Fritsch, Maniatis, Molecular Cloning - A Laboratory Manual, 1989, Cold Spring Harbor Laboratory Press, USA, sind grundlegende Verfahren zur in vitro Synthese von Nukleinsäuren angegeben.

Aus der WO 03/072809 A1 ist ein Verfahren zur selektiven Amplifikation mindestens einer Zielnukleinsäure in einer Probe bekannt, die mindestens eine Zielnukleinsäure und mindestens eine Nicht-Zielnukleinsäure enthält. Die Zielnukleinsäure weist einen geringem Schmelzpunkt als die Nicht-Zielnukleinsäure auf. Bei dem Verfahren werden ein oder mehrere Zyklen eines Nukleinsäuredenaturierungschritts von einem Amplifikationsschritt, bei dem mindestens ein Amplifikationsprimer eingesetzt wird, gefolgt. Der Denaturierungsschritt wird bei oder oberhalb der Schmelztemperatur der mindestens einen Zielnukleinsäure, aber unterhalb der Schmelztemperatur der mindestens einen Nicht-Zielnukleinsäure durchgeführt, so dass die Amplifikation der Nicht-Zielnukleinsäure im wesentlichen unterdrückt wird.

WO 2004/058987 offenbart unter anderem ein Verfahren zur Durchführung eines Strand- Displacement Verfahrens bei dem die DNA unter Verzicht eines Denaturierungsschrittes einem sogenannten whole genome strand displacement amplification - Verfahren unterzogen wird. Der Verzicht eines Denaturierungsschrittes führt zur Reduktion des sogenannten "Sequence bias" des amplifizierten Produktes.

Eine Strand-Displacement-Reaktion (SDR) ist eine Methode, bei der eine Polymerase-Reaktion mit Oligonukleotiden gestartet wird, wobei während der Reaktion ein Abschälen eines "alten" Stranges ("Strand-Displacement") einer doppelsträngigen Nukleinsäure von dem anderen "alten" (komplementären) Strang bewirkt wird, um ein Binden von Oligonukleotiden an den anderen "alten" Strang zu ermöglichen. Wichtig für die Reaktion ist die Initiierung, wobei man verschiedene Techniken unterscheiden kann:
(A) Eine Trennung der beiden hybridisierenden Nukleinsäure-Stränge (z.B. DNA-Stränge) kann durch eine Hitzedenaturierung bei 95°C erfolgen. Bei dieser Temperatur werden nachweislich die beiden DNA-Stränge voneinander getrennt, so dass Oligonukleotide an den denaturierten (d.h. voneinander getrennten) DNA-Strängen binden können. Die Initiation der SDR kann dann erfolgen (z.B. Protokoll des GenomiPhi-Kits, Amersham Biosciences GmbH, Freiburg i. Br., Deutschland). Dieses Verfahren hat jedoch einen wesentlichen Nachteil: Eine Erhitzung auf 95°C führt zu einer Schädigung der DNA, z.B. durch Depurinisierung oder Strangbrüche (Suzuki T., Ohsumi S., Makino, K. (1994), Mechanistic studies on depurination and apurinic site chain breakage in oligodeoxyribonucleotides, Nucleic Acid Res. 22(23): 4997-5003).
(B) Eine Trennung der beiden hybridisierenden Nukleinsäure-Stränge (DNA-Stränge) kann auch durch eine Alkali-Denaturierung (Protokoll des REPLI-g Kits, QIAGEN GmbH, Hilden, Deutschland) erfolgen. Dies hat jedoch den Nachteil, dass nach der Alkali-Zugabe neutralisiert werden muss. Dies bedeutet zusätzliche Pipettierschritte und eine Veränderung im Reaktionsmilieu.
(C) Eine nächste Methode versucht keine Strangtrennung zu erreichen, sondern verwendet Endonukleasen, um Einzelstrangbrüche einzufügen, an deren 3'-OH Ende die Polymerase-Reaktion starten kann (vergl. z.B. US 6,884,586).
(D) Eine vierte Methode schließlich verwendet keine Denaturierung, wie z.B. von Notomi T., Okayama H., Masubuchi H., Yonekawa T., Watanabe K., Amino N., Hase T. (2000), Loop-mediated isothermal amplification of DNA, Nucleic Acids Res. 15; 28(12):E63 beschrieben. Dies führt jedoch zu deutlich schlechteren Ergebnissen, da vermutlich die in der DNA enthalten Einzelstrangbrüche für die Verlängerung in einer SDR verwendet werden.

Aufgabe der vorliegenden Erfindung ist es daher, ein Verfahren zur Aktivierung einer Nukleinsäure, insbesondere einer Desoxyribonukleinsäure (DNA), für eine Strand-Displacement-Reaktion (Strangverdrängungsreaktion) anzugeben, das die oben beschriebenen Nachteile des Standes der Technik nicht aufweist. Diese Aufgabe löst die Erfindung durch ein Verfahren zur Aktivierung einer Nukleinsäure für eine Polymerase-Reaktion mit den Schritten:
(a) Erwärmen einer Nukleinsäure auf eine Temperatur von 60°C bis 65°C.
(b) Abkühlen der Nukleinsäure auf eine Temperatur von 4°C bis 45°C, bevorzugt 15°C bis 42°C, insbesondere 25°C bis 37°C, und
(c) Starten der Polymerase-Reaktion durch Zugabe einer Polymerase zu der Nukleinsäure.

Weitere vorteilhafte Ausgestaltungen der vorliegenden Erfindung sind in den Ansprüchen, der Beschreibung, den Beispielen und der Zeichnung angegeben.

Nachfolgend werden einige der verwendeten Begriffe näher erläutert.

Strand-Displacement-Reaktion (SDR): Unter Strand-Displacement-Reaktion wird hier jede Reaktion verstanden, bei der eine Polymerase verwendet wird, die eine Strand-Displacement Aktivität aufweist, oder bei der eine Reaktionsbedingung verwendet wird, die ein Strand-Displacement ermöglicht. Hierzu zählen z.B. die Strand-Displacement-Amplification (SDA) genauso wie die Multiple-Displacement-Amplification (MDA) oder die Rolling-Circle-Amplification (RCA) sowie alle Unterformen dieser Reaktionen, wie z.B. Restriction-aided RCA (RCA-RCA) oder MDA mit *nested* Primern, lineare und exponentielle Strand-Displacement-Reaktionen oder auch Helicase-Dependent Amplification (vergl. z.B. die europäischen Patentanmeldungen Nr. 20050112639, 20050074804, 20050069939 und 20050069938, sowie Wang G., Maher E., Brennan C., Chin L., Leo C., Kaur M., Zhu P., Rook M., Wolfe J.L., Makrigiorgos G.M. (2004), DNA amplification method tolerant to sample degradation, Genome Res. Nov;14(11):2357-2366; Milla M.A., Spears P.A., Pearson R.E., Walker G.T. (1998), Use of the restriction enzyme AvaI and ero-Bst polymerase in strand displacement amplification, Biotechniques Mar;24(3):392-396; Nagamine K., Watanabe K., Ohtsuka K., Hase T., Notomi T. (2001), Loop-mediated isothermal amplification reaction using a nondenatured template, Clin Chem. 47(9):1742-1743; Notomi et al 2001 (siehe oben); Lage J.M., Leamon J.H., Pejovic T., Hamann S., Lacey M., Dillon D., Segraves R., Vossbrinck B., Gonzalez A., Pinkel D., Albertson D.G., Costa J., Lizardi P.M. (2003), Whole genome analysis of genetic alterations in small DNA samples using hyperbranched strand displacement amplification and array-CGH, Genome Res.13(2):294-307; und Vincent M., Xu Y., Kong H. (2004), Helicase-dependent isothermal DNA amplification, EMBO Rep. 5(8):795-800).

Strand-Displacement-Polymerase: Zu den Strand-Displacement-Polymerasen gehören alle Polymerasen, die ein Strand-Displacement durchführen können. Hierzu zählen Enzyme, wie z.B. phi29-DNA-Polymerase, Cp-1-DNA-Polymerase, PRD1-DNA-Polymerase, phi15-DNA-Polymerase, phi21-DNA-Polymerase, PZE-DNA-Polymerase, PZA-DNA-Polymerase, Nf-DNA-Polymerase, M2Y-DNA-Polymerase, B103-DNA-Polymerase, SF5-DNA-Polymerase, GA-1-DNA-Polymerase, Cp-5-DNA-Polymerase, Cp-7-DNA-Polymerase, PR4-DNA-Polymerase, PR5-DNA-Polymerase, PR722-DNA-Polymerase, L17-DNA-Polymerase, Klenow DNA-Polymerase, Vent DNA Polymerase, Deep Vent DNA Polymerase, Bst DNA Polymerase, 9oNm™ DNA Polymerase, Polymerase III-Systeme und Bca DNA Polymerase. Die Strand-Dipslacement-Polymerasen können auch in mutierter Form vorliegen, z.B. als sogenannte exominus-Varianten (d.h. ohne Exonuklease Aktivität).

DNA: Desoxyribonukleinsäure (DNA) kommt natürlicherweise in Organismen vor, kann aber auch außerhalb von Organismen vorkommen oder diesem hinzugefügt worden sein. Die Länge der DNA kann unterschiedlich sein. Die DNA kann durch Veränderungen modifiziert sein. Die Basen der DNA können modifiziert sein. Die DNA kann Basenanaloga (z.B. auch non-Purin oder non-Pyrimidin Analoga) oder Nukleotidanaloga (z.B. PNA) enthalten. Die DNA kann Anhänge enthalten, wie z.B. Proteine oder Aminosäuren.

Die vorliegende Erfindung betrifft somit ein Verfahren zur Aktivierung einer Nukleinsäure (insbesondere einer doppelsträngigen DNA) für eine Strand-Displacement-Reaktion, wobei das Verfahren die folgenden Schritte umfasst: (a) Erwärmen der Nukleinsäure auf eine (im Vergleich zu herkömmlichen Verfahren moderate) Temperatur von 60°C bis 65°C; (b) Abkühlen der Nukleinsäure auf eine Temperatur von 4°C bis 45°C, bevorzugt 15°C bis 42°C, insbesondere 25°C bis 37°C; und (c) Starten der Strand-Displacement-Reaktion durch Zugabe einer Polymerase zu der Nukleinsäure. Die bei einem bisher verwendeten Verfahren eingesetzte hohe Temperatur von 95°C, die sich, wie oben beschrieben, insofern negativ auswirkt, als eine eingesetzte Nukleinsäure (z.B. DNA) erheblich durch Strangbrüche und Depurinisierung geschädigt wird, kann durch das erfindungsgemäbe Verfahren vermieden werden. Auch auf die bisher häufig angewandte Trennung doppelsträngiger Nukleinsäuren mittels Alkalibehandlung mit den entsprechenden nachteiligen Begleitumständen kann durch das neue Verfahren verzichtet werden. Das erfindungsgemäße Verfahren bietet somit eine die eingesetzte Nukleinsäure schonende Möglichkeit, eine Strand-Displacement-Reaktion vorzubereiten und durchzuführen. Das erfindungsgemäße Verfahren wird bevorzugt dann eingesetzt, wenn eine hitzelabile Polymerase zum Einsatz kommt. Im Falle der vorliegenden Erfindung wird eine Polymerase dann als hitzelabil bezeichnet, wenn diese nach einer Behandlung von 10 Minuten bei einer Temperatur von 65°C nur noch eine Aktivität von maximal 20 % der Ausgangsaktivität aufweist, d.h. wenn die Polymerase zu mindestens 80 % inaktiviert worden ist.

Die oben genannte Variante wird bevorzugt dann eingesetzt, wenn eine hitzelabile Polymerase zum Einsatz kommt.

Die moderate Temperatur, bei der Zellen oder isolierte DNA erhitzt werden, liegt zwischen 60°C und 65°C, bevorzugt bei 65°C. Die Erhitzung der DNA oder der Zellen kann beispielsweise direkt im SDR-Reaktionsgemisch erfolgen.

Die Erfindung beschreibt somit eine Aktivierung einer Nukleinsäure (insbesondere DNA) fur eine Strand-Displacement Reaktion durch einen moderaten Erhitzungsschritt. Die erfindungsgemäße Methode umfasst bei Verwendung von isolierter Nukleinsäure (DNA) und einer hitzelabilen Strand-Displacement-Polymerase dementsprechend folgende Teilschritte: (1) Die Nukleinsäure (DNA) wird auf eine moderat hohe Temperatur erhitzt. (2) Die Nukleinsäure (DNA) wird abgekühlt, wobei die Temperatur nach dem Abkühlvorgang maximal eine Höhe haben darf, bei der die Polymerase noch nicht deutlich an Aktivität verliert. Die Nukleinsäure wird daher auf eine Temperatur von 4°C bis 45°C abgekühlt, besonders bevorzugt auf einen Bereich von 15°C bis 42°C und ganz besonders bevorzugt auf einen Bereich von 25°C und 37°C. (3) Die SDR-Reaktion wird durch Zugabe der (hitzelabilen) Polymerase gestartet.

Die oben vorgestellte Variante wird bevorzugt dann eingesetzt, wenn eine hitzelabile Polymerase verwendet wird.

Das erfindungsgemäße Verfahren kann aber nicht nur für reine bzw. gereinigte DNA verwendet werden, sondern auch für DNA, die noch in einem Zellverbund enthalten ist. Die Methode für die Verwendung von DNA, die noch im Zellverbund enthalten ist, und einer hitzelabilen Strand-Displacement-Polymerase hat dementsprechend folgende Teilschritte: (1) Die DNA-haltigen Zellen werden auf eine moderat hohe Temperatur erhitzt. (2) Die DNA-haltigen Zellen werden abgekühlt, wobei die Temperatur nach dem Abkühlvorgang maximal eine Höhe haben darf, bei der die Polymerase noch nicht deutlich an Aktivität verliert. (3) Die SDR-Reaktion wird durch Zugabe der Polymerase gestartet.

Bei beiden Methoden kann die hitzelabile Strand-Displacement-Polymerase auch durch eine hitzestabile Strand-Displacement-Polymerase ersetzt werden. Dann kann der Teilschritt (1) jeweils direkt mit der Polymerase durchgeführt werden.

In der Zeichnung zeigen
- Fig. 1: die Ausbeute der Reaktionen aus Beispiel 1;
- Fig. 2: die Ct-Werte der Real-time PCR aus Beispiel 1;
- Fig. 3: die Ausbeute der Reaktionen aus Beispiel 2;
- Fig. 4: die Ct-Werte der Real-time PCR aus Beispiel 2;
- Fig. 5: die Ct-Werte der Real-time PCR aus Beispiel 3;
- Fig. 6: die Ausbeute der Reaktionen aus Beispiel 4; und
- Fig. 7: die Ct-Werte der Real-time PCR aus Beispiel 4.
- Fig. 8: die Ct-Werte der Real-Time PCR aus Beispiel 5.
- Fig. 9: die Ct-Werte der Real-Time PCR des Locus 11/12 aus Beispiel 6.
- Fig. 10: die Ct-Werte der Real-Time PCR des Locus 665 aus Beispiel 6.

Die Erfindung wird nachfolgend anhand von Beispielen näher erläutert.

### Beispiel 1

Das Beispiel soll zeigen, dass durch einen einfachen Temperaturaktivierungsschritt eine SDR (hier eine Multiple-Displacement-Amplification, MDA) ausgehend von Vollblut ermöglicht wird, die hinsichtlich der DNA-Ausbeute und DNA-Qualität vergleichbar zu einer Reaktion nach dem Stand der Technik (Kontrollreaktion) ist.

Erfindungsgemäße Reaktion: Die MDA-Reaktion wurde mit den REPLI-g Reagenzien (QIAGEN GmbH, Hilden, Deutschland) durchgeführt. Jeweils 0,5, 1 und 2 µl Vollblut, stabilisiert durch EDTA oder Citrat, wurden mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und anschließend auf 65°C erwärmt. Nach Abkühlen auf Raumtemperatur (ca. 20-25°C) wurde die DNA-Polymerase aus dem REPLI-g Kit zugegeben. Die Reaktion wurde dann für 6 h bei 30°C durchgeführt.

Kontroll-Reaktion nach dem Stand der Technik: Die MDA-Reaktion wurde mit den REPLI-g Reagenzien (QIAGEN) durchgeführt: 0,5 µl Vollblut, stabilisiert durch EDTA, wurden mit 2,5 µl phosphatgepufferter Salzlösung (PBS, aus dem REPLI-g Kit von QIAGEN) versetzt. Anschließend wurden 3,5 µl des frisch angesetzten Denaturierungspuffers (360 mM KOH, 9 mM EDTA, 100 mM DTT) hinzu gegeben und für 10 min auf Eis inkubiert. Das Gemisch wurde nach der Inkubation mit Solution B (REPLI-g Kit) neutralisiert. Das Gemisch wurde mit 12,5 µl 4x Reaction Mix aus dem REPLI-g Kit (dieser enthält Oligonukleotide mit einer Zufallssequenz, Reaktionspuffer und dNTPs) und dest. Wasser auf ein Volumen von 49,5 µl aufgefüllt. Daraufhin wurde 0,5 µl der DNA-Polymerase aus dem REPLI-g Kit zugegeben. Die Reaktion wurde dann für 6 h bei 30°C durchgeführt.

Nach Ablauf der Reaktion wurde die DNA-Konzentration mit PicoGreen nach Hersteller-Protokoll (Molecular Probes Inc., Eugene, Oregon, USA) gemessen. 10 ng der MDA-DNA wurde für eine Real-time PCR (Polymerasekettenreaktion) eingesetzt. 4 verschiedene Loci wurden auf ihre Repräsentanz im Amplifikat hin untersucht:
(a) eine Sequenz, die als Sat bezeichnet wurde
   (Primer-Sequenzen:
   Sat1.1 TCTTTCCACTCCATTGCAT und
   Sat1.2 GGAATGGAATCAACCCAA
(b) eine Sequenz aus dem β-Aktin Gen
   Primer 1 GTCTCAAGTCAGTGTACAGG
   Primer 2 GTGATAGCATTGCTTTCGTG
(c) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG) und
(d) eine Sequenz, die aus dem als "699" bezeichneten Locus entstammt
   (Probe: TGAACTGCTCCTTGGCAGGGATTT,
   Primer 1: TGCTCCCTGTCCCATCTG,
   Primer 2: AGACAGTATGCCTTTATTTCACCC).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal messbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis von Beispiel 1 lässt sich wie folgt zusammenfassen: (1) Die Ausbeute aus den Reaktionen, bei denen die DNA erfindungsgemäß durch einen 65°C-Schritt aktiviert wurde, ist vergleichbar zur Ausbeute der Kontroll-Reaktion. (2) Auch die Repräsentanz der Sequenzen in der amplifizierten DNA ist vergleichbar, wenn 0,5 µl Blut eingesetzt wird. Lediglich die Sequenz des Sat-Locus ist niedriger als in der Kontroll-Reaktion. (3) Größere Volumina als 0,5 µl wirken hemmend auf die MDA Reaktion mit 65°C Aktivierung. Dies ist erkennbar an den höheren Ct-Werten (d.h. schlechtere Repräsentanz der Sequenzen in der amplifizierten DNA).

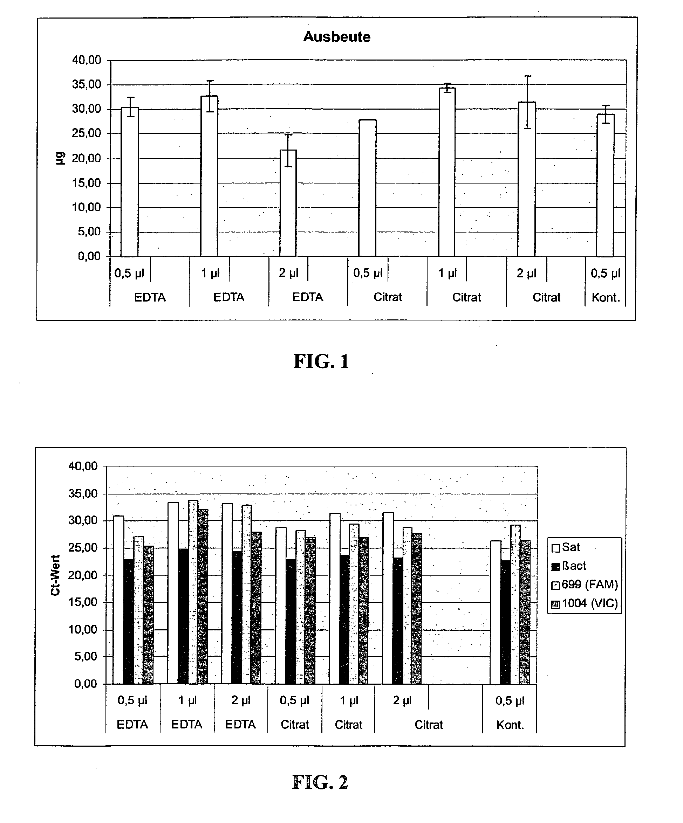

Die Ausbeute der Reaktionen aus Beispiel 1 ist in Fig. 1 graphisch dargestellt. Fig. 2 zeigt die Ct-Werte der Real-time PCR aus Beispiel 1.

### Beispiel 2

Dieses Beispiel dient dazu zu zeigen, dass zu geringe Temperaturen bei einem Temperaturaktivierungsschritt die Qualität der DNA, die während der SDR entsteht, beeinträchtigen kann.

Erfindungsgemäße Reaktion: Die MDA-Reaktion wurde mit den REPLI-g Reagenzien (QIAGEN) durchgeführt: 0,5 µl Vollblut, stabilisiert durch EDTA, wurde mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und durch verschiedene Temperaturen aktiviert (30, 40, 45, 50, 55, 60 bzw. 65°C). Nach Abkühlen auf Raumtemperatur (ca. 20-25°C) wurde die DNA-Polymerase aus dem REPLI-g Kit zugegeben. Die Reaktion wurde für 6 h bei 30°C durchgeführt.

Kontroll-Reaktion nach dem Stand der Technik: Die MDA-Reaktion wurde mit den REPLI-g Reagenzien (QIAGEN) durchgeführt: 0,5 µl Vollblut, stabilisiert durch EDTA, wurden mit 2,5 µl PBS (REPLI-g Kit) versetzt. Anschließend wurden 3,5 µl des frisch angesetzten Denaturierungspuffer (360 mM KOH, 9 mM EDTA, 100 mM DTT) hinzu gegeben und für 10 min auf Eis inkubiert. Das Gemisch wurde nach der Inkubation mit Solution B (REPLI-g Kit) neutralisiert. Das Gemisch wurde dann mit 12,5 µl 4x Reaction Mix aus dem REPLI-g Kit (dieser enthält Oligonukleotide mit einer Zufallssequenz, Reaktionspuffcr und dNTPs) und dest. Wasser auf ein Volumen von 49,5 µl aufgefüllt. Daraufhin wurde 0,5 µl der DNA-Polymerase aus dem REPLI-g Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurden für eine Real-time PCR eingesetzt. 2 verschiedene Loci wurden auf ihre Repräsentanz im Amplifikat hin untersucht:
(a) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG und
   Primer 2: AGCAGAATTCTGCACATGACG) sowie
(b) eine Sequenz, die aus dem als "699" bezeichneten Locus entstammt
   (Probe: TGAACTGCTCCTTGGCAGGGATTT,
   Primer 1: TGCTCCCTGTCCCATCTG,
   Primer 2: AGACAGTATGCCTTTATTTCACCC).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal messbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis von Beispiel 2 lässt sich wie folgt zusammenfassen: (1) Die Ausbeute aus den Reaktionen, bei denen die DNA durch einen 30 bis 65°C-Schritt aktiviert wurde, ist vergleichbar zur der Kontroll-Reaktion. (2) Auch die Repräsentanz der Sequenzen in der amplifizierten DNA ist nur etwas schlechter als bei der Kontrollreaktion, wenn die Aktivierung bei 60 oder 65°C durchgeführt wurde. Bei einer Aktivierung der DNA für die Strand-Displacement Reaktion unter 60°C wird die Repräsentanz der betrachteten Sequenzen schlechter.

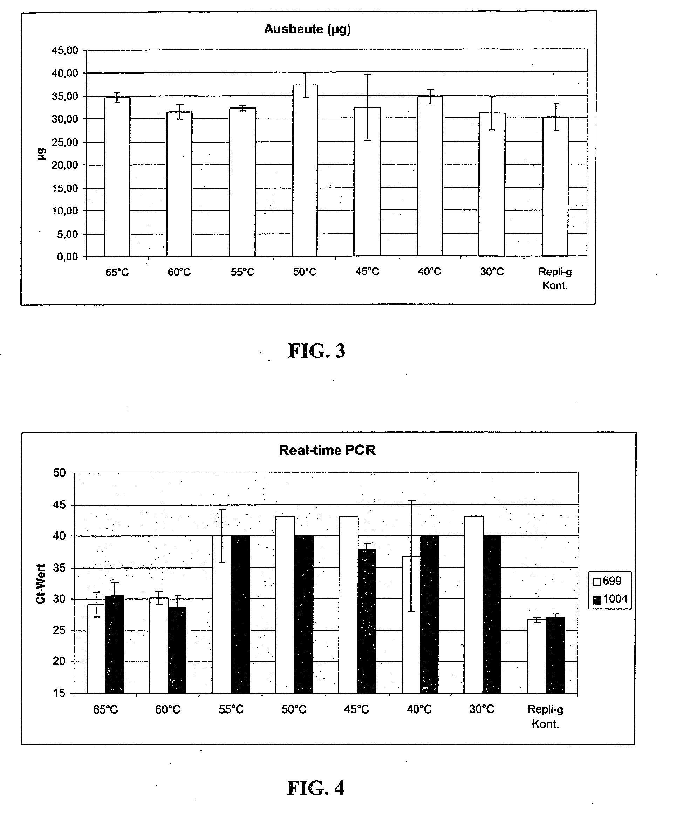

Die Ausbeute der Reaktionen aus Beispiel 2 ist in Fig. 3 graphisch dargestellt. Fig. 4 zeigt die Ct-Werte der Real-Time PCR aus Beispiel 2.

### Beispiel 3

Mit diesem Beispiel soll gezeigt werden, dass innerhalb bestimmter Temperaturgrenzen eine einfache Temperaturaktivierung vor der SDR die Qualität der in der SDR entstehenden DNA im Vergleich zu einer Reaktion nach dem Stand der Technik (Kontrollreaktion) nicht beeinträchtigt.

Erfindungsgemäße Reaktion: Die MDA-Reaktion wurde mit den REPLI-g Reagenzien (QIAGEN) durchgeführt. 0,5 µl Vollblut, stabilisiert durch EDTA, wurden mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und durch verschiedene Temperaturen aktiviert (60, 65 und 70°C). Nach Abkühlen des Reaktionsansatzes auf Raumtemperatur (ca. 20-25 °C) wurde die DNA-Polymerase aus dem REPLI-g Kit zugegeben. Die Reaktion wurde für 6 h bei 30°C durchgeführt.

Kontroll-Reaktion nach dem Stand der Technik: Die MDA-Reaktion wurde mit den REPLI-g Reagenzien (QIAGEN) durchgeführt.: 0,5 µl Vollblut, stabilisiert durch EDTA, wurden mit 2,5 µl PBS (REPLI-g Kit) versetzt. Anschließend wurden 3,5 µl des frisch angesetzten Denaturierungspuffers (360 mM KOH, 9 mM EDTA, 100 mM DTT) hinzu gegeben und für 10 min auf Eis inkubiert. Das Gemisch wurde nach der Inkubation mit Solution B (REPLI-g Kit) neutralisiert. Das Gemisch wurde mit 12,5 µl 4x Reaction Mix aus dem REPLI-g Kit (dieser enthält Oligonukleotide mit einer Zufallssequenz, Reaktionspuffer und dNTPs) und mit dest. Wasser auf ein Volumen von 49,5 µl aufgefüllt. Daraufhin wurden 0,5 µl der DNA-Polymerase aus dem REPLI-g Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurden für eine Real-time PCR eingesetzt. 4 verschiedene Loci wurden auf ihre Repräsentanz im Amplifikat hin untersucht:
(a) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG) und
(b) eine Sequenz, die aus dem als "699" bezeichneten Locus entstammt
   (Probe: TGAACTGCTCCTTGGCAGGGATTT,
   Primer 1: TGCTCCCTGTCCCATCTG,
   Primer 2: AGACAGTATGCCTTTATTTCACCC).
(c) eine Sequenz, die als Sat bezeichnet wurde
   (Primer-Sequenzen:
   Sat1.1 TCTTTCCACTCCATTGCAT und
   Sat1.2 GGAATGGAATCAACCCAA
(d) eine Sequenz aus dem β-Aktin Gen
   Primer 1 GTCTCAAGTCAGTGTACAGG
   Primer 2 GTGATAGCATTGCTTTCGTG

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal meßbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis lässt sich wie folgt zusammenfassen. Die Repräsentanz der Sequenzen in der amplifizierten DNA ist bei dem erfindungsgemäßen Ansatz teilweise besser als bei der Kontrollreaktion, wenn die Aktivierung bei 60°C, 65°C oder 70°C durchgeführt wurde.

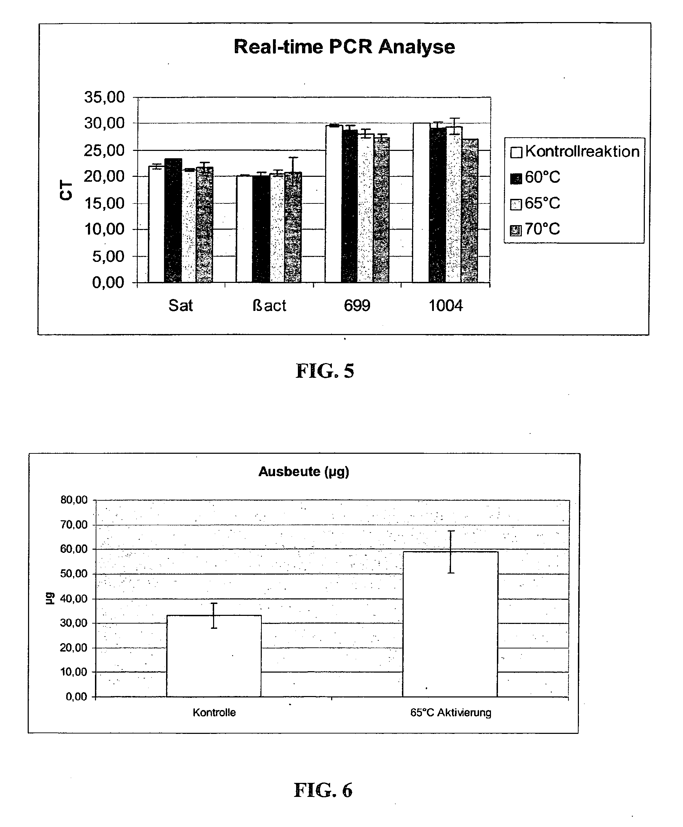

Fig. 5 zeigt die Ct-Werte der Real-Time PCR-Analyse von DNA aus der SDR-Reaktion von Beispiel 3.

### Beispiel 4

Dieses Beispiel dient dazu zu zeigen, dass durch einen einfachen Temperaturaktivierungsschritt eine SDR (hier eine Multiple Displacement Amplification, MDA), ausgehend von isolierter genomischer DNA, ermöglicht wird, die hinsichtlich DNA-Ausbeute und DNA-Qualität vergleichbar zu einer Reaktion nach dem Stand der Technik (Kontrollreaktion) ist.

Erfindungsgemäße Reaktion: Die MDA-Reaktion wurde mit den REPLI-g Reagenzien (QIAGEN) durchgeführt. 2,5 µl einer Lösung von genomischer DNA aus humanen Zellen (Konzentration: 4 ng/µl) wurden mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und durch einen Inkubationsschritt bei 65°C aktiviert. Nach Abkühlen des Reaktionsansatzes auf Raumtemperatur (ca. 20-25 °C) wurde die DNA-Polymerase aus dem REPLI-g Kit zugegeben. Die Reaktion wurde für 6 h bei 30°C durchgeführt.

Kontroll-Reaktion nach dem Stand der Technik: Die MDA-Reaktion wurde mit den REPLI-g Reagenzien (QIAGEN) durchgeführt. 2,5 µl genomische DNA aus humanen Zellen (Konzentration: 4 ng/µl) wurden mit 2,5 µl frisch angesetztem Denaturierungspuffer (50 mM KOH, 1,25 mM EDTA) versetzt und für 3 min bei Raumtemperatur inkubiert. Das Gemisch wurde nach der Inkubation mit einer 1:10 Verdünnung der Solution B (REPLI-g Kit) neutralisiert. Das Gemisch wurde mit 12,5 µl 4x Reaction Mix aus dem REPLI-g Kit (dieser enthält Oligonukleotide mit einer Zufallssequenz, Reaktionspuffer und dNTPs) und dest. Wasser auf ein Volumen von 49,5 µl aufgefüllt. Daraufhin wurden 0,5 µl der DNA-Polymerase aus dem REPLI-g Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurde für eine Real-time PCR eingesetzt. 1 Locus wurde auf die Repräsentanz im Amplifikat hin untersucht:
(a) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal messbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis war, dass die Ausbeute aus den Reaktionen, bei denen die DNA durch einen 65°C-Schritt aktiviert wurde, fast doppelt so hoch war wie bei der Kontrolle nach dem Stand der Technik. Die Repräsentanz der Sequenzen in der amplifizierten DNA ist in der erfindungsgemäßen Probe und der Kontrollprobe vergleichbar.

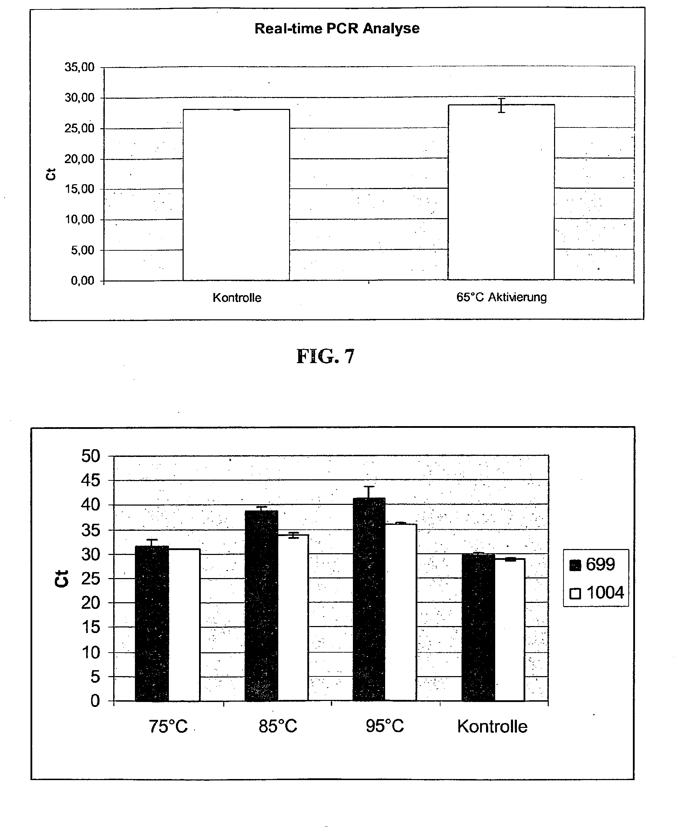

Die Ausbeute der Reaktionen aus Beispiel 4 ist in Fig. 6 graphisch dargestellt. Fig. 7 zeigt die Ct-Werte der Real-Time PCR aus Beispiel 4.

### Beispiel 5

In diesem Beispiel wird gezeigt, dass durch einen einfachen Denaturierungsschritt bei zunehmend hohen Temperaturen bestimmte Sequenzen nicht mehr in einer SDR (hier eine Mutliple Displacement Amplification) amplifiziert werden können.

Test-Reaktionen: Die MDA-Reaktion wurde mit den REPLI-g Reagenzien (QIAGEN) durchgeführt: 10 ng isolierte DNA wurde bei Temperaturen von 75°C, 85°C oder 95°C für 10 min. inkubiert. Alternativ wurde in Kontrollreaktionen eine alkalische Denaturierung in einem KOH-Puffer durchgeführt. Nach der chemischen Denaturierung mit KOH wurde die Lösung neutralisiert, um die MDA Reaktionsbedingungen nicht zu beeinflussen. Anschließend an die Hitzebehandlung oder chemische Denaturierung wurden der DNA-haltigen Lösung 12,5 µl 4x Reaction Mix (dieser enthält Oligonukleotide mit einer Zufallssequenz, Reaktionspuffer und dNTPs) und Wasser auf ein Volumen von 39,5 µl aufgefüllt. Anschließend wurde den Reaktionsgemischen die DNA-Polymerase aus dem REPLI-g Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurde für eine Real-time PCR eingesetzt. 2 verschiedene Loci wurden auf ihre Repräsentanz im Amplifikat hin untersucht:
(a) eine Sequenz, die aus dem als 1004 bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG) und
(b) Sequenz, die aus dem als 699 bezeichneten Locus entstammt
   (Probe:TGAACTGCTCCTTGGCAGGGATTT,
   Primer 1: TGCTCCCTGTCCCATCTG,
   Primer 2: AGACAGTATGCCTTTATTTCACCC).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal messbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2fach höheren DNA-Ausgangsmenge im Testansatz gegenüber dem Kontrollansatz für die gemessene Sequenz.

Ergebnis:
(1) Mit zunehmender Temperatur bei der Hitzebehandlung der DNA wurden immer höhere CT-Werte gemessen.
(2) Beide Loci verhalten sich unterschiedlcih auf die Hitzebehandlung: Im Falle des Locus 699 konnte eine Ct-Verschiebung von 9,6 Zyklen gemessen werden, vergleicht man die Werte bei einer 75°C bzw. 95°C Behandlung. Im Falle des Locus 1004 immerhin noch eine Ct-Verschiebung von 5 Zyklen gemessen werden. D.h. mit zunehmender Temperatur der Hitzebehandlung der DNA wurden im MDA Amplifikationsprodukt die gemessenen Loci immer schlechter nachgewiesen, obwohl in einer Einheitsmenge des Amplifikationsproduktes (10ng) für Real-time PCR eingesetzt wurde.

Eine Ct-Verschiebung von 9,6 bzw. 5 Zyklen entspricht einer 780fachen^{*} bzw. 30 fachen^{*} geringeren Repräsentation der Sequenz 699 bzw. 1004 im Amplifikationsprodukt der DNA, die vor Amplifikation auf 95°C erhitzt wurde, verglichen zu einer bei 75°C behandelten DNA (^{*} diese Werte legen zugrunde, dass in jedem Zyklus der real-time PCR eine Verdopplung stattfindet).

Fig. 8 zeigt die Ct-Werte der Real-Time PCR aus Beispiel 5.

### Beispiel 6

Dieses Beispiel zeigt, bei welchen Temperaturen ein optimaler Temperaturaktivierungsschritt erfolgt, um eine möglichst gute Sequenz-Repräsentation bei einer SDR (hier eine Mutliple Displacement Amplification) zu erzielen.

Test-Reaktionen: Die MDA-Reaktion wurde mit den REPLI-g Reagenzien (QIAGEN) durchgeführt: 10 ng isolierte DNA wurde bei verschiedenen Temperaturen für 5 min inkubiert. Alternativ wurde in Kontrollreaktionen DNA in einem Mehrschrittverfahren bestehend aus (1) Zugabe von KOH, (2) Inkubation der DNA in der KOH Lösung für 5 min und (3) Neutralisierung der alkalischen Lösung für die REPLI-g Reaktion bereitet.

Anschließend an die Hitzebehandlung oder chemische Denaturierung wurden der DNA-haltigen Lösung in einer REPLI-g Reaktion amplifiziert. Die Reaktion wurde für 8 h bei 33°C durchgeführt.

Nach Ablauf der Reaktion wurde die DNA-Konzentration mit PicoGreen nach Hersteller-Protokoll (Molecular Probes) gemessen. 10 ng der MDA-DNA wurde für eine Real-time PCR eingesetzt. 2 verschiedene Loci wurden auf ihre Repräsentanz im Amplifikat hin untersucht:
(a) eine Sequenz, die aus dem als 11/12 bezeichneten Locus entstammt
   (Primer 1: TTTCTGTAACAGCTAAGGAC,
   Primer 2: TAGGGTGCTTAGCTGTTAAC) und
(b) Sequenz, die aus dem als 665 bezeichneten Locus entstammt
   (Primer 1: CTCTTGCTCAGCCTATATAC,
   Primer 2: GTAGAAAATGTAGCCCATTAC.

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal messbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2fach höheren DNA-Ausgangsmenge im Testansatz gegenüber dem Kontrollansatz für die gemessene Sequenz.

Ergebnis:
(1) Mit zunehmender Temperatur bei der Hitzebehandlung der DNA wurden immer höhere CT-Werte gemessen.
(2) Die niedrigsten CT-Werte (also die beste Repräsentation der hier untersuchten Loci) ergibt sich bei einer thermischen Vorbehandlung bei einer Temperatur von 65°C bis 85°C.
(3) Bei diesen Loci wurden bei den Temperaturen 65-85°C bessere Ct-Werte gemessen als bei der Referenz-Behandlung mittels KOH.
(4) Durch den Vergleich Beispiel 5, bei dem bei 85°C bereits schlechtere CT-Werte gemessen wurden als bei der Referenzbehandlung mittels KOH, kann gefolgert werden, dass die optimale Behandlungstemperatur abhängig ist vom Locus im Genom einer Zelle.

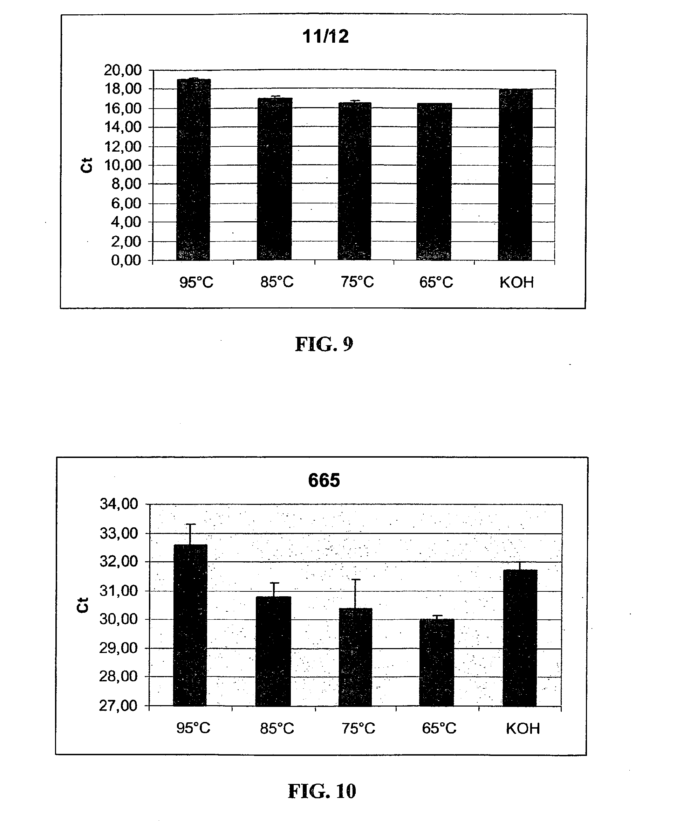

Fig. 9 zeigt die Ct-Werte der Real-Time PCR des Locus 11/12 aus Beispiel 6. Fig. 10 zeigt die Ct-Werte der Real-Time PCR des Locus 665 aus Beispiel 6.

## Patentansprüche

1. Verfahren zur Aktivierung einer doppelsträngigen DNA für eine Strand-Displacement-Reaktion mit den Schritten:
a) Erwärmen einer DNA auf eine Temperatur von 60°C bis 65°C',
b) Abkühlen der DNA auf eine Temperatur von 4°C bis 45°C, bevorzugt 15°C bis 42°C, insbesondere 25°C bis 37°C,
c) Starten der Strand-Displacement-Reaktion durch Zugabe einer hitzelabilen Polymerase zu der DNA,
wobei in den Verfahrensschritten a), b) und/ c) keine Alkalibehandlung erfolgt.

2. Verfahren nach Anspruch 1, **dadurch gekennzeichnet, dass** die Strand-Displacement-Reaktion eine Multiple-Displacement-Reaktion ist.

3. Verfahren nach einem der vorhergehenden Ansprüche, **dadurch gekennzeichnet, dass** die DNA in Schritt a) auf eine Temperatur von 65°C erwärmt wird.

4. Verfahren nach einem der vorhergehenden Ansprüche, **dadurch gekennzeichnet, dass** die DNA in gereinigter Form in einer wässrigen Lösung vorliegt.

5. Verfahren nach einem der Ansprüche 1 bis 3, **dadurch gekennzeichnet, dass** die DNA in einer Zelle vorliegt.

## Claims

1. Method for activating a double-stranded DNA for a strand-displacement reaction having the steps:
a) warming a DNA to a temperature of 60°C to 65°C,
b) cooling the DNA to a temperature of 4°C to 45°C, preferably 15°C to 42°C, in particular 25°C to 37°C,
c) starting the strand-displacement reaction by adding a heat-labile polymerase to the DNA,
wherein no alkali treatment proceeds in the process steps a), b) and c).

2. Method according to Claim 1, **characterized in that** the strand-displacement reaction is a multiple-displacement reaction.

3. Method according to any one of the preceding claims, **characterized in that** the DNA is warmed in step a) to a temperature of 65°C.

4. Method according to any one of the preceding claims, **characterized in that** the DNA is present in purified form in an aqueous solution.

5. Method according to any one of Claims 1 to 3, **characterized in that** the DNA is present in a cell.

## Revendications

1. Procédé pour l'activation d'un ADN double brin pour une réaction de déplacement de brin, comportant les étapes suivantes :
a) chauffage d'un ADN à une température de 60 à 65°C,
b) refroidissement de l'ADN à une température de 4 à 45°C, de préférence de 15 à 42°C, en particulier de 25 à 37°C,
c) démarrage de la réaction de déplacement de brin par addition d'une polymérase thermolabile à l'ADN, aucun traitement par une base n'étant effectué dans les étapes a), b) et c).

2. Procédé selon la revendication 1, **caractérisé en ce que** la réaction de déplacement de brin est une réaction de déplacement multiple.

3. Procédé selon l'une des revendications précédentes, **caractérisé en ce que** l'ADN est, dans l'étape a), chauffé à une température de 65°C.

4. Procédé selon l'une des revendications précédentes, **caractérisé en ce que** l'ADN se présente sous forme purifiée en solution aqueuse.

5. Procédé selon l'une des revendications 1 à 3, **caractérisé en ce que** l'ADN se trouve dans une cellule.
